# EUROPEAN PATENT SPECIFICATION

(11) **EP 0 462 933 B1**
(45) Date of publication and mention of the grant of the patent: **08.06.1994**
(21) Application number: 91810459.7
(22) Date of filing: 17.06.1991
(51) Int. Cl.: C07C 335/12, A61K 31/17

(54) **Novel N-benzyl-N1-phenyl and -phenalkyl-thioureas**
N-Benzyl-N1-phenyl und -phenalkyl-thioharnstoffe
N-benzyl-N1-phényl et -phénalkyl-thiourées

(30) Priority: 18.06.1990 GB 9013575; 22.06.1990 GB 9013953
(43) Date of publication of application: 27.12.1991
(73) Proprietor: SANDOZ LTD., 4002 Basel (CH); SANDOZ-PATENT-GMBH, 79539 Lörrach (DE); SANDOZ-ERFINDUNGEN Verwaltungsgesellschaft m.b.H., 1235 Wien (AT); SANDOZ PRODUCTS LIMITED, Horsforth Leeds LS18 4RP (GB)
(72) Inventor: Walpole, Christopher Simon John, London W9 3JS (GB); Wrigglesworth, Roger, Sevenoaks, Kent (GB)

(56) References cited:
- EP-A- 0 068 590
- EP-A- 0 282 127
- GB-A- 2 206 347
- GB-A- 2 226 313

## Description

The present invention relates to novel thioureas having pharmaceutical properties, to processes for their production, pharmaceutical compositions comprising them and their use as pharmaceuticals.

More particularly the present invention relates to compounds of formula I 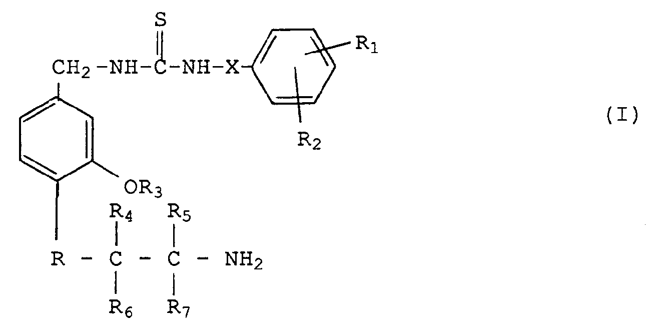
wherein:
- one of R₁ and R₂: is branched C₄alkyl and the other is hydrogen or branched C₄alkyl,
- R₃: is hydrogen or C₁₋₄alkyl,
- R₄ and R₅: are, independently, hydrogen, halogen, C₁₋₅alkyl, substituted C₁₋₅alkyl, substituted or unsubstituted aryl or a group of formula -COOH, -COOR₈ or -CONR₉R₁₀, wherein R₈ is C₁₋₅alkyl and R₉ and R₁₀ are, independently, hydrogen or C₁₋₅alkyl,
- R₆ and R₇: have, independently, the meanings given for R₄ and R₅ or, together with the carbon atoms to which they are attached, form a substituted or unsubstituted C₃₋₇cycloalkyl group,
- R: is -O-, -S- or -NH- and
- X: is -(CH₂)ₙ- wherein n is zero, 1, 2 or 3,

physiologically hydrolysable and acceptable esters or amides thereof and pharmaceutically acceptable salts of such compounds esters and amides.

Alkyl groups as and alkyl moieties of R₃ through R₁₀ may be branched or straight chain. Preferably such alkyl groups and moieties are straight chain.

By halogen is meant fluorine, chlorine, bromine or iodine.

Preferred aryl groups as R₄ to R₇ are phenyl and naphthyl. Such groups may be unsubstituted or substituted. Preferred substituents are selected from halogen, hydroxy, amino and carboxy. Especially preferred aryl groups as R₄ to R₇ are phenyl and substituted phenyl.

In formula I, the following significances are preferred either individually or in any combination or sub-combination:
1. R₁ is tertiary butyl. Preferably R₁ is in the 3- or 4-position. Most preferably R₁ is tertiary butyl in the 3- or 4-position.
2. R₂ is hydrogen or tertiary butyl. When R₁ is in the 3-position, R₂ is preferably tertiary butyl and is preferably in the 5-position. When R₁ is in the 4-position, R₂ is preferably hydrogen.
3. R₃ is hydrogen or methyl, preferably methyl.
4. R₄ to R₇, independently are hydrogen, methyl, ethyl, C₁₋₅alkyl substituted by OH or NH₂, phenyl or C₁₋₅alkyl-phenyl. Preferably at least one of R₄ to R₆ is hydrogen. More preferably at least three of R₄ to R₇, e.g. R₄, R₅ and R₆, are hydrogen. Most preferably all of R₄ to R₇ are hydrogen.
5. R is -O-.
6. X is -(CH₂)ₙ-, wherein n is 1 or 2, especially 1.

An especially preferred group of compounds of formula I are those wherein X has the meanings given under 6 above and especially such compounds wherein R₁ and R₂ are as defined under 1 and 2 above. A further preferred group of compounds of formula I are those wherein X has the meanings given under 6 above and each of R₄ to R₇ is hydrogen, especially such compounds wherein R₁ and R₂ are as defined under 1 and 2 above.

By the term "physiologically hydrolysable and acceptable esters or amides" as used herein is meant esters (e.g. of compounds of formula I wherein one or more of R₄ to R₇ is -COOH and/or R₃ is hydrogen) or amides which are hydrolysable under physiological conditions to produce acids or alcohols which are themselves non-toxic, i.e. have no significant undesirable side effects at desired dosage levels. Such esters include esters with mono- and di-carboxylic acids, e.g. esters of the compounds of formula I in which the 3-hydroxy group is acetylated and esters with lower, e.g. C₁₋₄ alkanols. Such amides include those derived from organic carboxylic acids, e.g. acetic acid amides, including amino acid amides, e.g. glycine amides.

The compounds, esters, amides and salts of the present invention are within the ambit of the invention defined and disclosed for the first time in UK Patent Application No 89 28581.1 (= CA-A-2006115, published June 23, 1990, and UK Patent Publication No. 2 226 313, published June 27, 1990) and defined and/or subsequently disclosed in corresponding applications world-wide. The compounds, esters, amides and salts of the invention comprise a formally novel group. Compounds, esters, amides and salts of the invention also exhibit increased oral potency as compared with compounds, esters, amides and slats disclosed in the said UK Patent Application.

The present invention also provides a process for the production of the compounds, amides, esters and salts of the invention which process comprises:
a) for the production of a compound of formula I, deprotecting a compound of formula I which is in amino protected form;
b) for the production of a physiologically hydrolysable and acceptable ester or amide of a compound of formula I, esterifying or amidating a compound of formula I, for example in which R₃ is hydrogen, by reaction with an appropriate acylating or amidating agent;

and recovering a compound of formula I, ester or amide, thus obtained in free or pharmaceutically acceptable salt form.

Process step a) may be carried out in accordance with standard techniques for the removal of amino protecting groups as known in the art. Suitable amino protecting groups include p-toluene sulphonyl, benzyl, formyl and trifluoroacetyl. An especially preferred embodiment of process step (a) comprises deprotection of a compound of formula II 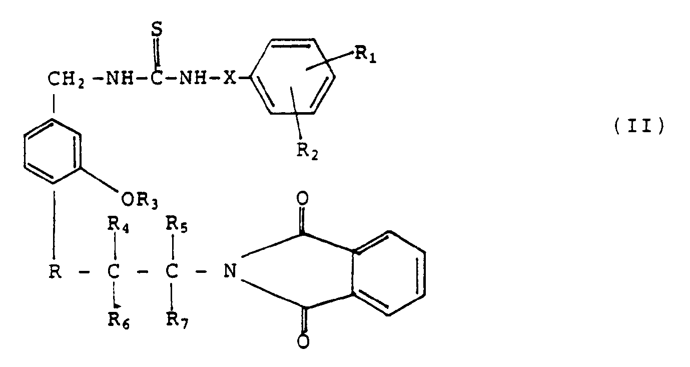
wherein R, R₁ to R₇ and X have the meanings given above, by reaction with hydrazine. This reaction may be carried out in analogy with known methods, for example, by reaction of the compound of formula II with hydrazine hydrate in an inert solvent or diluent, e.g. alkane/alkanol, at ambient or elevated temperatures, e.g. at a temperature of from 0°C to reflux.

Process step b) may also be performed employing conventional acylation or amidation methods, e.g. by reaction with an appropriate, e.g. C₁₋₄, acyl halide or anhydride.

Starting materials required for process step (a), e.g. compounds of formula II, may be produced by the following reaction scheme in which R, R₁ to R₇ and X are as defined above and Y is halogen, e.g. bromine.

The intermediates of formulae VI, V, IV, III and II in the reaction scheme are also formally novel, and also form part of the present invention per se.

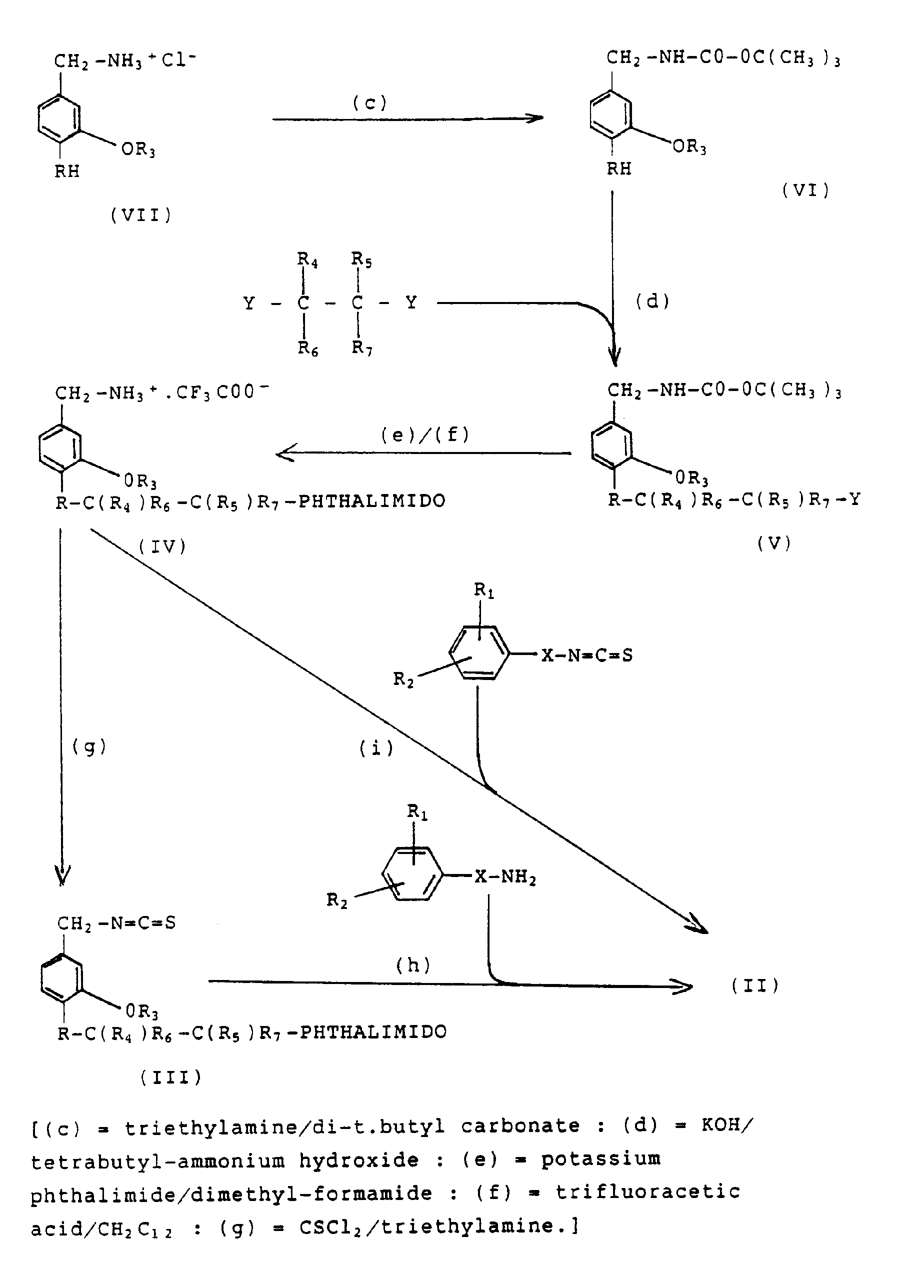

The compounds of formula I as defined above may exist in free or salt form. Suitable pharmaceutically acceptable salts of compounds of formula I, e.g. wherein one or more of R₄ to R₇ is -COOH for use in accordance with the present invention include, for example, the sodium, potassium and calcium salts as well as quaternary ammonium, e.g. triethylammonium salts. Such salts also in particular include pharmaceutically acceptable acid addition salts, e.g. salts with inorganic or organic acids such as hydrochloric acid, acetic acid, fumaric acid, citric acid and benzoic acid.

The following Examples are illustrative of the process of the present invention.

The following abbreviations are used:
- DMF: = dimethylformamide
- TFA: = trifluoroacetate
- TLC: = thin layer chromatography
- HPLC: = high pressure liquid chromatography

HPLC retention times in the following examples are measured on a C-18 Microbondapak reverse phase column using the following gradient and conditions: (solution A = 0.1% CF₃C00H, solution B = CH₃CN, flow rate = 2.25ml/min. constant) : O min. - 90% A, 10% B; 2 min. - 90% A, 10% B; 6 min. - 45% A, 55% B; 22 min. - 40% A, 60% B; 29 min. - 0% A, 100% B; 31 min. - 90% A, 10% B.

### EXAMPLE 1:

N-[4-(2-aminoethoxy)-3-methoxybenzyl]-N′-[3,5-di(t-butyl)benzyl]thiourea : [Formula I : R = (CH₃)₃C-in the 3-position; R₂ = (CH₃)₃C- in the 5-position; X = -CH₂-; R₃ = CH₃-; R = 0; R₄, R₅, R₆, R₇ all = H.]

### Process step (a).

1.8g N-[4-(2-phthalimidoethoxy)-3-methoxybenzyl]-N′-[3,5-di(t-butyl)-benzyl]thiourea are suspended in 50ml ethanol and heated to approximately 60°C, until the solution becomes homogeneous. 0.14 ml 1-hexene and 0.73 ml 64% hydrazine hydrate are added and the heating continued for 2 hours. The reaction is shown to be complete by TLC. The mixture is cooled until a thick white precipitate forms and transferred to a separating funnel. 50 ml t-butylmethylether, 25 ml water, 25 ml 1N NaOH and 25 ml 50% NaOH are added and the resulting mixture shaken thoroughly. The organic layer is extracted twice as above and the combined aqueous layers extracted once with 150 ml t-butyl methyl ether. The combined organic phases are washed with brine, dried over Na₂SO₄, filtered and the solvent removed in vacuo. The residual oil is purified via flash column chromatography. (CH₂Cl₂ : methanol / 10:1) elutes the less polar components and methanol the product. The product is obtained as a colourless glass after drying in vacuo (30°C, 0.1 mmHg).
TLC MeOH; Rf = 0.25.
¹HNMR [CDCl₃, 200 MHz]:δ 1.25(18H,s,Ar(t-butyl)); 2.62(2H,s,broad,NH₂); 3.04(2H,t,J=5Hz, CCH₂N); 3.74(3H,s,ArOCH₃); 3.94(2H,t,J=5Hz,OCH₂C); 4.55 (4H,s,broad,benzylCH₂); 6.71(2H,s,broad, 2xNH); 6.75(3H,m,vanillylArH); 7.09(2H,s,ArH); 7.32(1H,s,ArH).
HPLC Retention time = 9.82 min

Following the steps of the reaction scheme, the starting material may be prepared as follows:

### c) t-Boc-vanillylamine

18.0g Vanillylamine hydrochloride and 10.6g triethylamine are dissolved in 250ml water and placed in a 1L round bottomed flask. 20.5g di-t-butyldicarbonate in 200ml dioxan are added with stirring over a period of 15 minutes. The resulting mixture is stirred overnight at room temperature.

The dioxan is removed in vacuo and the aqueous residue extracted with CHCl₃ (5x10ml). The combined extracts are dried over MgSO₄, filtered and the solvent removed in vacuo to leave a brown oil which is purified via flash column chromatography (cyclohexane : ethylacetate / 5:2) to give a colourless oil which crystallises on standing. [TLC cyclohexane : ethylacetate / 1:1; Rf. = 0.5]

### d) t-Boc-(4-(2-bromoethoxy)-3-methoxybenzylamine

21.0g t-Boc-vanillylamine, 250ml 1,2-dibromoethane, 66ml 40% KOH and 6.6ml 40% tetrabutylammonium hydroxide are combined in a 500ml round bottomed flask and the resulting mixture is heated at 50°C for 3 hours with rapid stirring.

The mixture is cooled, diluted with 200ml CH₂Cl₂, washed with water (3x200ml) and the combined aqueous washings extracted once with 600ml CH₂Cl₂. The combined organic layers are washed with brine, dried over MgSO₄, filtered and the solvent removed in vacuo to leave a white solid which is used without further purification. [TLC cyclohexane : ethylacetate / 1:1; Rf. = 0.6]

### e) t-Boc-(4-(2-phthalimidoethoxy)-3-methoxybenzylamine

23.0g t-Boc-(4-(2-bromoethoxy)-3-methoxybenzylamine and 11.8g potassium phthalimide are suspended in 500ml dry DMF in a round bottomed flask. The resulting suspension is heated at 50°C for 2 hours with rapid stirring. After 30 mins the mixture becomes homogeneous. The mixture is then cooled and the DMF removed under high vacuum. The resulting solid residue is purified via flash column chromatography (cyclohexane : ethylacetate / 1:1) to give a white solid. [TLC cyclohexane : ethylacetate / 1:1; Rf. = 0.45.

### f) 4-(2-phthalimidoethoxy)3-methoxybenzylamine.TFA

26.5g t-Boc-(4-(2-phthalimidoethoxy)-3-methoxybenzylamine are dissolved in 200ml CH₂Cl₂ in a 500ml round bottomed flask. 15ml trifluoroacetic acid are added dropwise with stirring. On completion of the addition, the mixture is stirred for a further 2 hours at room temperature (the reaction is shown to be completed by the loss of starting material (TLC, cyclohexane : ethylacetate / 1:1)). The solvent is removed in vacuo, initially on the water pump and then at high vacuum. The resulting colourless oil solidifies on standing and is used without further purification.

### g) 4-(2-phthalimidoethoxy)-3-methoxybenzylisothiocyanate

5.0g 4-(2-phalimidoethoxy)-3-methoxybenzylamine.TFA are dissolved/suspended in 250 ml water in a round bottomed flask. 0.9 ml thiophosgene in 200 ml CH₂Cl₂ are added and the mixture stirred rapidly. A few crystals of phenolphthalein are added, then 1M NaOH added dropwise until the pink colour in the aqueous layer is constant.

The organic layer is separated off, washed with 150 ml water, dried over Na₂SO₄, filtered and the solvent removed in vacuo to leave a yellow solid. The solid residue is purified by flash column chromatography (cyclohexane) to give a pale yellow solid. TLC cyclohexane : ethylacetate / 1:1; Rf. = 0.85; MS m/z 368 (M⁺)

### h) N-[4-(2-phthalimidoethoxy)-3-methoxybenzyl]-N′-[3,5-di(t-butyl)benzyl]thiourea

0.85g 3,5-di(t.-butyl)benzylamine are dissolved in 25 ml dry ethylacetate under an atmosphere of N₂ and 1.6g 4-(2-phthalimidoethoxy)-3-methoxybenzylisothiocyanate in 20 ml dry EtOAc are added dropwise with stirring over 1 hour. The resulting solution is then allowed to stir at room temperature overnight. The solvent is removed in vacuo to give a pale yellow solid which is used without purification. TLC cyclohexane : ethyalcetate / 1:1; Rf. = 0.25
¹HNMR [CDCl₃:200MHz]δ 1.3(18H,s,Ar(t-butyl)); 3.65(3H,s,ArOCH₃); 4.1 (2H,m,CCH₂N); 4.25(2H,m,0CH₂C); 4.55(4H,s,benzylCH₂); 6.1(2H,s,broad,NH); 6.8(3H,m,vanillylArH); 7.2(3H,m,ArH); 7.8(4H,m,ArH); MS m/z 601 (M⁺)

### EXAMPLE 2:

N-[4-(2-aminoethoxy)-3-methoxybenzyl]-N′-[4-t-butylbenzyl)]thiourea : [Formula I : R₁ = (CH₃)₃C- in the 4-position; R₂, R₄, R₅, R₆, R₇ all = H; X = -CH₂-; R₃ = CH₃-; R = -0-]

### Process step (a)

1.5g N-[4-(2-phthalimidoethoxy)-3-methoxybenzyl]-N′[4-t-butyl-benzyl]thiourea are dissolved/suspended in 200ml ethanol and heated to ca. 60°C. 0.01 ml 1-hexene and 0.1ml 64% aqueous NH₂NH₂. H₂0 are added and heating continued for 3 hrs. The reaction mixture is concentrated to ca. 100ml and shaken with 50ml t-butylmethyl ether, 50ml H₂0, 1ml Na0H and 2ml 50% Na0H. The organic layer is extracted 2x in this manner and the combined aqueous extracts extracted 1x with 200ml t-butylmethyl ether. The combined organic phases are dried over Na₂S0₄, filtered and the solvent removed by evaporation in vacuo. The residue is purified by flash column chromatography, initially with CH₂Cl₂: methanol (10:1) to elute less polar components, followed by methanol to yield the title compound as a colourless glass.
1H-NMR [CDCl₃, 400MHz] δ 1.30(9H,s,Ar-t-butyl); 1.54(2H,s,broad,NH₂); 3.08(2H,t,J=5Hz,CCH₂N); 3.80(3H,s,ArOCH₃); 4.00(2H,t,J=5Hz,OCH₂C); 4.54(2H,s,broad,benzylCH₂); 4.59(2H,s,broad,benzylCH₂); 6.25(2H,s,broad CSNH); 6.76(3H,m,vanillyl ArH); 7.2(2H,d,J=8Hz,ArH); 7.33 (2H,d,J = 8Hz, ArH)
TLC MeOH; Rf. = 0.2. HPLC Retention time : 9.26 min.

The HCl salt may be obtained from the free base by reaction with HCl in solution in methanol. The salt is re-crystallised from 50% aqueous ethanol: m.p.= 125-130°C : TLC (silica, CHCl₃/C₂H₅0H/CH₃C00H 120:90:5), Rf = 0.7.

The starting material is prepared as follows:

### i) N-[4-(2-Phthalimidoethoxy)-3-methoxy-benzyl]-N′-[-t-butyl-benzyl]thiourea

0.93g t-butylbenzylisothiocyanate in 50ml tetrahydrofuran are added dropwise under N₂ over a period of 15 mins. to a stirred solution of 2.0g of the product of example 1f) and 0.46g triethylamine in 50ml dry tetrahydrofuran. The obtained reaction mixture is then stirred for 2 hrs. at room temperature, the solvent evaporated in vacuo the residue suspended in 100ml H₂0 and extracted 3x with 100ml CH₂Cl₂. The combined organic extracts are dried over Na₂S0₄, filtered and the solvent removed in vacuo. The residue is purified by flash column chromatography to yield the title compound. TLC (cyclohexane: ethylacetate/1:1) Rf = 0.3.

The compounds amides esters and pharmaceutically acceptable salts of the invention (hereinafter collectively : COMPOUNDS OF THE INVENTION) have pharmaceutical, in particular analgesic and anti-inflammatory, activity as indicated in standard test models, for example as described hereinafter.

### 1. TAIL-FLICK TEST IN THE MOUSE

The method is based on that of D'Amour et al. J. Pharmacol. Exp. Ther. 72, 74-79 (1941), but employing unstarved mice (male + female, 16-25 g). Animals are divided into control and test groups, control animals receiving a vehicle injection only. Each test animal is placed in an individual perspex cylinder to prevent movement with its tail protruding along a narrow groove. The tail of each animal is exposed to a beam of radiant heat at ca. 35 mm from the tail root, from a lamp of known output and temperature, place directly under the tail. Test substance is administered s.c. or p.o. 30 mins. post introduction into the cylinder. The time in seconds taken by the mouse to flick its tail out of the light beam is recorded 30 to 15 mins prior to administration of test substance. Animals whose reaction times differ by more than 25 % are discarded. Reaction time is re-determined 15 and 30 mins post administration. Extension of reaction time by > 75 % over mean pre-treatment values in the same animal are taken as indicative of analgesic response. Three doses are employed per test substance and 10 animals per dose. ED₅₀ values (95 % confidence limits) are estimated in accordance with the method of Litchfield and Wilcoxon and represent the dose prolonging treatment reaction time by > 75 % in 50 % of test animals.

COMPOUNDS OF THE INVENTION are active in the above test model at dosages of the order of from about 0.1 to about 50.0 »M/kg, s.c. and from about 0.5 to about 75.0 »M/kg, p.o..

### 2. YEAST INDUCED INFLAMMATION TEST IN THE MOUSE

20 »l, 20 % fresh yeast suspension is injected into the plantar region of one hind paw and saline is injected into the other. Degree of inflammation is estimated by the relative increase in paw weight (yeast injected v.s. saline) 2 hrs. post-injection. Test substance is administered s.c. or p.o. at varying dosage at the same time as yeast/saline treatment. 5 animals are used/dose and testing at each dose is repeated 2 - 3 times, control and test values being compared statistically as above. ED₅₀ values are taken as the dosage required to effect 50 % inhibition of inflammation as compared with control animals not receiving test substance, and are established from dose response curves plotting % inflammation vs. dose.

COMPOUNDS OF THE INVENTION are active in the above test model at dosages of the order of from about 0.05 to 25.0»M/kg, s.c. and from about 0.5 to about 75.0»M/kg, p.o..

COMPOUNDS OF THE INVENTION are accordingly indicated for use as pharmaceuticals, e.g. as analgesics for the treatment of pain of various genesis or aetiology, for example dental pain and headache, particularly vascular headache, such as migraine, cluster headache, and mixed vascular syndromes as well as nonvascular, tension headache, and as anti-inflammatory agents for the treatment of inflammatory diseases or conditions, for example the treatment of arthritis and rheumatic diseases, Raynaud's disease, inflammatory bowel disorders, trigeminal or herpetic neuralgia, inflammatory eye disorders (e.g. uveitis) psoriasis and cystitis as well as other chronic inflammatory conditions.

Having regard to their analgesic/anti-inflammatory profile they are, in particular, indicated for use in the treatment of inflammatory pain, for the treatment of hyperalgesia and, in particular, the treatment of severe chronic pain, e.g. for the treatment of deafferentation pain as an alternative to surgical procedures.

According to a further embodiment, COMPOUNDS OF THE INVENTION are also indicated for use in the prophylactic or curative treatment of asthma, of epithelial tissue damage or dysfunction, e.g. spontaneous lesions, of herpes simplex, and in the control of disturbances of visceral motility at respiratory, genitourinary, gastrointestinal or vascular level. COMPOUNDS OF THE INVENTION are thus indicated, e.g. for treating wounds, burns, allergic skin reactions, pruritus and vitiligo, for the prophylactic or curative treatment of gastrointestinal disorders such as gastric ulceration, duodenal ulcers and diarrhoea, for the prophylactic or curative treatment of gastric lesions induced by necrotising agents, for example ethanol, for the treatment of vasomotor or allergic rhinitis and for the treatment of bronchial disorders or bladder disorders.

For the above uses the required dosage will of course vary depending on the mode of administration, the particular condition to be treated and the effect desired. An indicated daily dosage for analgesic and/or anti-inflammatory use is in the range of from about 2 to about 500mg p.o., e.g. from about 5 or 50 to about 100mg p.o.. whereby, within the above ranges, for anti-inflammatory use lower dosages will generally be appropriate than for analgesic use. Indicated daily dosages are conveniently administered once, in divided dosages 2 to 4 times/day, or in sustained release or retard form. Dosage forms suitable for oral administration accordingly comprise from about 0.5 to about 500mg, e.g. from about 1.25 to about 50 or 100mg COMPOUND OF THE INVENTION admixed with an appropriate solid or liquid, pharmaceutically acceptable, diluent or carrier therefor.

In accordance with the foregoing the present invention also provides:
A. A COMPOUND OF THE INVENTION for use as a pharmaceutical, for example for use in a method:
   i) for the treatment of pain of various genesis or aetiology, for example in relation to the treatment of any specific disease or condition as hereinbefore set forth;
   ii) for the treatment of inflammatory diseases and/or inflammatory pain, for example in relation to the treatment of any specific disease or condition as hereinbefore set forth;
   iii) for the treatment (including prophylaxis) of asthma, epitheleal tissue damage or dysfunction, gastrointestinal disorder or gastric lesion induced by necrotising agents;
   iv) for the control of disturbance of visceral motility at respiratory, genitourinary, gastrointestinal or vascular level; or
   v) for the treatment of rhinitis or bronchial or bladder disorder;
B. A COMPOUND OF THE INVENTION for use in the preparation of a pharmaceutical composition for use in a method as defined under any one of A(i) to A(v) above; as well as
C. A pharmaceutical composition comprising a COMPOUND OF THE INVENTION together with a pharmaceutically acceptable diluent or carrier therefor.

## Claims (Claims for the following Contracting State(s): AT, BE, CH, DE, FR, GB, IT, LI, LU, NL, SE)

1. A compound of formula I 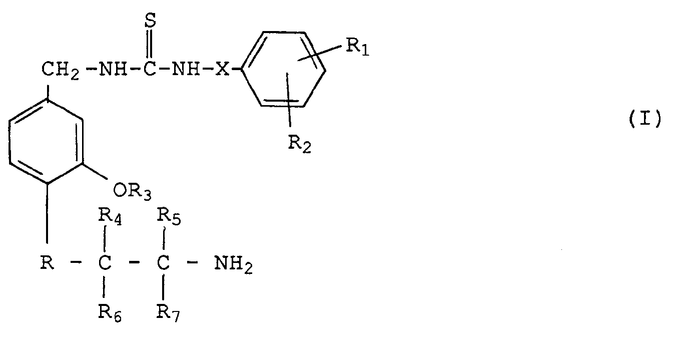 wherein one of
R₁ and R₂ is branched C₄alkyl and the other is hydrogen or branched C₄alkyl,
R₃ is hydrogen or C₁₋₄alkyl,
R₄ and R₅ are, independently, hydrogen, halogen, C₁₋₅alkyl, substituted C₁₋₅alkyl, aryl, substituted or unsubstituted aryl or a group of formula, -COOH, -COOR₈ or -CONR₉R₁₀, wherein R₈ is C₁₋₅alkyl and R₉ and R₁₀ are, independently, hydrogen or C₁₋₅alkyl,
R₆ and R₇ have, independently, the meanings given for R₄ and R₅ or, together with the carbon atom to which they are attached, form a substituted or unsubstituted C₃₋₇cycloalkyl group,
R is -O-, -S- or -NH- and
X is (CH₂)ₙ- wherein n is zero, 1, 2 or 3,
or physiologically hydrolysable and acceptable ester or amide thereof or pharmaceutically acceptable salt of such a compound, ester or amide.

2. A compound, ester, amide or salt according to claim 1 wherein, in formula I, R₁ is t-butyl and R₂ is hydrogen or t-butyl.

3. A compound, ester, amide or salt according to claim 2 wherein, in formula I, R₁ is t-butyl in the 3-position and R₂ is t-butyl in the 5-position or R₁ is t-butyl in the 4-position and R₂ is hydrogen.

4. A compound, amide or salt according to any one of claims 1 to 3 wherein, in formula I, R₃ is methyl; R₄, R₅, R₆ and R₇ are each hydrogen, R is -0- and X is -CH₂-.

5. A compound or salt according to any one of claims 1 to 4.

6. N-[4-(2-aminoethoxy)-3-methoxy benzyl]-N¹-[3,5-di(t-butyl)-benzyl]thiourea or a pharmaceutically acceptable acid addition salt thereof.

7. N-[4-(2-aminoethoxy)-3-methoxy benzyl]-N¹-[4-t.butyl-benzyl]thiourea or a pharmaceutically acceptable acid addition salt thereof.

8. A compound, ester, amide or salt as defined in any one of claims 1 to 6 for use as a pharmaceutical.

9. A compound, ester, amide or salt as defined in claim 7 for use in the treatment of pain, inflammatory disease or inflammatory pain.

10. A pharmaceutical composition comprising a compound, ester, amide or salt as defined in any one of claims 1 to 7, together with a pharmaceutically acceptable diluent or carrier therefor.

## Claims (Claims for the following Contracting State(s): ES, GR)

1. Process for the production of a compound of formula I 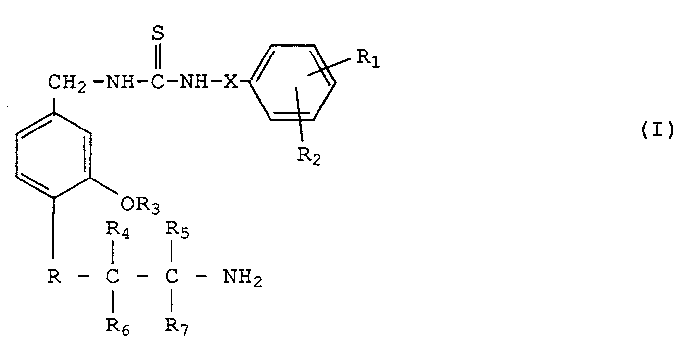 wherein one of
R₁ and R₂ is branched C₄alkyl and the other is hydrogen or branched C₄alkyl,
R₃ is hydrogen or C₁₋₄alkyl,
R₄ and R₅ are, independently, hydrogen, halogen, C₁₋₅alkyl, substituted C₁₋₅alkyl, aryl, substituted or unsubstituted aryl or a group of formula, -COOH, -COOR₈ or -CONR₉R₁₀, wherein R₈ is C₁₋₅alkyl and R₉ and R₁₀ are, independently, hydrogen or C₁₋₅alkyl,
R₆ and R₇ have, independently, the meanings given for R₄ and R₅ or, together with the carbon atom to which they are attached, form a substituted or unsubstituted C₃₋₇cycloalkyl group,
R is -O-, -S- or -NH- and
X is -(CH₂)ₙ- wherein n is zero, 1, 2 or 3,
or physiologically hydrolysable and acceptable ester or amide thereof or pharmaceutically acceptable salt of such a compound, ester or amide,
which process comprises
a) for the production of a compound of formula I, deprotecting a compound of formula I which is in amino protected form; or
b) for the production of a physiologically hydrolysable and acceptable ester or amide of a compound of formula I, esterifying or amidating a compound of formula I by reaction with an appropriate acylating or amidating agent;
and recovering a compound of formula I, ester or amide, thus obtained in free or pharmaceutically acceptable salt form.

2. Process according to claim 1 wherein, in formula I, R₁ is t-butyl and R₂ is hydrogen or t-butyl.

3. Process according to claim 2 wherein, in formula I, R₁ is t-butyl in the 3-position and R₂ is t-butyl in the 5-position or R₁ is t-butyl in the 4-position and R₂ is hydrogen.

4. Process according to any one of claims 1 to 3 wherein, in formula I, R₃ is methyl; R₄, R₅, R₆ and R₇ are each hydrogen, R is -0- and X is -CH₂-.

5. Process according to claim 1 wherein, in formula I,
R₁ is t.-butyl in the 3-position,
R₂ is t.-butyl in the 5-position,
R₃ is methyl
R₄, R₅, R₆ and R₇ are each hydrogen,
R is -0- and
X is -CH₂-.

6. Process according to claim 1 wherein, in formula I,
R₁ is t.-butyl in the 4-position,
R₂ is hydrogen,
R₃ is methyl
R₄, R₅, R₆ and R₇ are each hydrogen,
R is -0- and
X is -CH₂-.

7. A compound of formula I as illustrated in claim 1 wherein R₁ to R₇, R and X have the meanings given in any one of claims 1 to 6 or physiologically hydrolysable and acceptable ester or amide thereof or pharmaceutically acceptable salt of such a compound, ester or amide, whenever prepared by a process as claimed in any one of claims 1 to 6.

8. A compound of formula I as illustrated in claim 1 wherein R₁ to R₇, R and X have the meanings given in any one of claims 1 to 6 or physiologically hydrolysable and acceptable ester or amide thereof or pharmaceutically acceptable salt of such a compound, ester or amide, or compound, ester, amide or salt as defined in claim 7 for use as a pharmaceutical.

9. A pharmaceutical composition comprising a compound of formula I as illustrated in claim 1 wherein R₁ to R₇, R and X have the meanings given in any one of claims 1 to 6 or physiologically hydrolysable and acceptable ester or amide thereof or pharmaceutically acceptable salt of such a compound, ester or amide, or compound, ester, amide or salt as defined in claim 7, together with a pharmaceutically acceptable solvent or carrier therefor.

## Patentansprüche (Patentansprüche für folgende(n) Vertragsstaat(en): AT, BE, CH, DE, FR, GB, IT, LI, LU, NL, SE)

1. Verbindung der Formel I 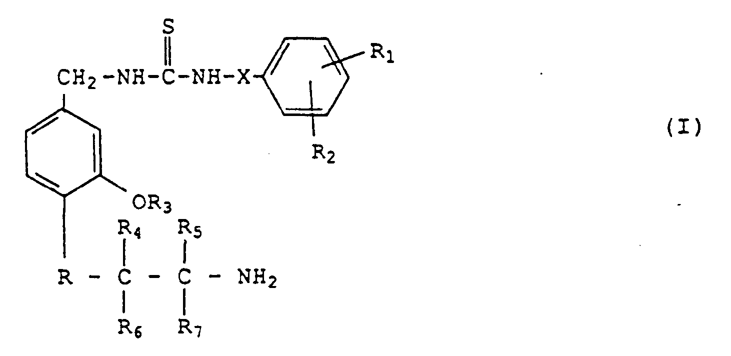 worin
einer der Reste R₁ und R₂ ein verzweigter C₄-Alkylrest ist und der andere Wasserstoff oder ein verzweigter C₄-Alkylrest ist,
R₃ Wasserstoff oder ein C₁-C₄-Alkylrest ist,
R₄ und R₅ unabhängig voneinander Wasserstoff, Halogen-, C₁-C₅-Alkyl-, substituierte C₁-C₅-Alkyl-, Aryl-, substituierte oder unsubstituierte Arylreste oder eine Gruppe der Formel -COOH, -COOR₈ oder -CONR₉R₁₀ sind, worin R₈ ein C₁-C₅-Alkylrest ist und R₉ und R₁₀ unabhängig voneinander Wasserstoff oder C₁-C₅-Alkylreste sind,
R₆ und R₇ unabhängig voneinander die für R₄ und R₅ angegebenen Bedeutungen haben oder zusammen mit dem Kohlenstoffatom, an das sie gebunden sind eine substituierte oder unsubstituierte C₃-C₇-Cycloalkylgruppe bilden,
R -O-, -S- oder -NH- ist und
X -(CH₂)ₙ- ist, worin n 0, 1, 2 oder 3 ist,
oder ein physiologisch hydrolysierbarer und annehmbarer Ester oder ein physiologisch hydrolysierbares und annehmbares Amid davon oder ein pharmazeutisch annehmbares Salz einer solchen Verbindung, eines solchen Esters oder Amids.

2. Verbindung, Ester, Amid oder Salz nach Anspruch 1 worin in Formel I R₁ ein t-Butylrest ist und R₂ Wasserstoff oder ein t-Butylrest ist.

3. Verbindung, Ester, Amid oder Salz nach Anspruch 2, worin in Formel I R₁ ein t-Butylrest in Position 3 ist und R₂ ein t-Butylrest in Position 5 ist oder R₁ ein t-Butylrest in Position 4 ist und R₂ Wasserstoff ist.

4. Verbindung, Amid oder Salz nach einem der Ansprüche 1 bis 3, worin in Formel I R₃ ein Methylrest ist, R₄, R₅, R₆ und R₇ jeweils Wasserstoff sind, R -O- ist und X -CH₂- ist.

5. Verbindung oder Salz nach einem der Ansprüche 1 bis 4.

6. N-[4-(2-Aminoethoxy)-3-methoxybenzyl]-N¹-[3,5-di(t-butyl)-benzyl]thioharnstoff oder ein pharmazeutisch annehmbares Säureadditionssalz davon.

7. N-[4-(2-Aminoethoxy)-3-methoxybenzyl]-N¹-[4-t-butylbenzyl]thioharnstoff oder ein pharmazeutisch annehmbares Säureadditionssalz davon.

8. Verbindung, Ester, Amid oder Salz nach einem der Ansprüche 1 bis 6 zur Verwendung als Pharmazeutikum.

9. Verbindung, Ester, Amid oder Salz nach Anspruch 7 zur Verwendung zur Behandlung von Schmerzen, entzündlichen Erkrankungen oder Entzündungsschmerzen.

10. Pharmazeutische Zusammensetzung enthaltend eine Verbindung, einen Ester, ein Amid oder Salz nach einem der Ansprüche 1 bis 7 zusammen mit einem pharmazeutisch annehmbaren Verdünnungsmittel oder Träger dafür.

## Patentansprüche (Patentansprüche für folgende(n) Vertragsstaat(en): ES, GR)

1. Verfahren zur Herstellung einer Verbindung der Formel I 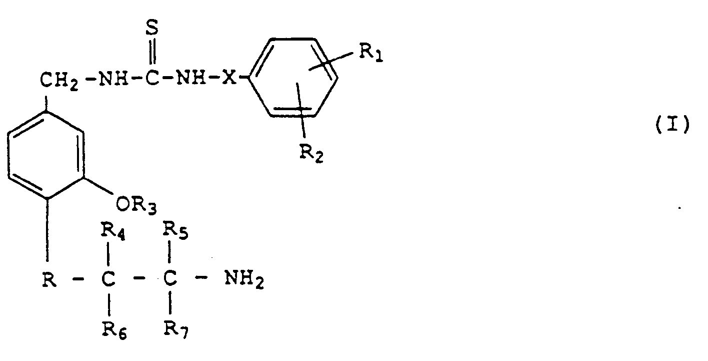 worin
einer der Reste R₁ und R₂ ein verzweigter C₄-Alkylrest ist und der andere Wasserstoff oder ein verzweigter C₄-Alkylrest ist,
R₃ Wasserstoff oder ein C₁-C₄-Alkylrest ist,
R₄ und R₅ unabhängig voneinander Wasserstoff, Halogen-, C₁-C₅-Alkyl-, substituierte C₁-C₅-Alkyl-, Aryl-, substituierte oder unsubstituierte Arylreste oder eine Gruppe der Formel -COOH, -COOR₈ oder -CONR₉R₁₀ sind, worin R₈ ein C₁-C₅-Alkylrest ist und R₉ und R₁₀ unabhängig voneinander Wasserstoff oder C₁-C₅-Alkylreste sind,
R₆ und R₇ unabhängig voneinander die für R₄ und R₅ angegebenen Bedeutungen haben oder zusammen mit dem Kohlenstoffatom, an das sie gebunden sind eine substituierte oder unsubstituierte C₃-C₇-Cycloalkylgruppe bilden,
R -O-, -S- oder -NH- ist und
X -(CH₂)ₙ- ist, worin n 0, 1, 2 oder 3 ist,
oder ein physiologisch hydrolysierbarer und annehmbarer Ester oder ein physiologisch hydrolysierbares und annehmbares Amid davon oder ein pharmazeutisch annehmbares Salz einer solchen Verbindung, eines solchen Esters oder Amids, wobei das Verfahren umfaßt, daß man
a) zur Herstellung einer Verbindung der Formel I bei einer Verbindung der Formel I, die in aminogeschützter Form vorliegt, die Schutzgruppen abspaltet oder
b) zur Herstellung eines physiologisch hydrolysierbaren und annehmbaren Esters oder Amids einer Verbindung der Formel I eine Verbindung der Formel I verestert oder amidiert durch Reaktion mit einem geeigneten Acylierungs- oder Amidierungsmittel;
und eine Verbindung der Formel I, einen Ester oder ein Amid gewinnt, die in freier Form oder in Form des pharmazeutisch annehmbaren Salzes erhalten wird.

2. Verfahren nach Anspruch 1, worin in Formel I R₁ ein t-Butylrest ist und R₂ Wasserstoff oder ein t-Butylrest ist.

3. Verfahren nach Anspruch 2, worin in Formel I R₁ ein t-Butylrest in Position 3 ist und R₂ ein t-Butylrest in Position 5 ist oder R₁ ein t-Butylrest in Position 4 ist und R₂ Wasserstoff ist.

4. Verfahren nach einem der Ansprüche 1 bis 3, worin in Formel I R₃ ein Methylrest ist; R₄, R₅, R₆ und R₇ jeweils Wasserstoff sind, R -O- ist und X -CH₂- ist.

5. Verfahren nach Anspruch 1, worin in Formel I R₁ ein t-Butylrest in Position 3 ist, R₂ ein t-Butylrest in Position 5 ist, R₃ ein Methylrest ist, R₄, R₅, R₆ und R₇ jeweils Wasserstoff sind, R -O- ist und X -CH₂- ist.

6. Verfahren nach Anspruch 1, worin in Formel I R₁ ein t-Butylrest in Position 4 ist, R₂ Wasserstoff ist, R₃ ein Methylrest ist, R₄, R₅, R₆ und R₇ jeweils Wasserstoff sind, R -O- ist und X -CH₂- ist.

7. Verbindung der Formel I, wie in Anspruch 1 dargestellt, worin R₁ bis R₇, R und X die in einem der Ansprüche 1 bis 6 angegebenen Bedeutungen haben oder ein physiologisch hydrolysierbarer und annehmbarer Ester oder ein physiologisch hydrolysierbares und annehmbares Amid davon oder ein pharmazeutisch annehmbares Salz einer solchen Verbindung, eines solchen Esters oder Amids, hergestellt mit einem Verfahren nach einem der Ansprüche 1 bis 6.

8. Verbindung der Formel I, wie in Anspruch 1 dargestellt, worin R₁ bis R₇, R und X die in einem der Ansprüche 1 bis 6 angegebenen Bedeutungen haben oder ein physiologisch hydrolysierbarer und annehmbarer Ester oder ein physiologisch hydrolysierbares und annehmbares Amid davon oder ein pharmazeutisch annehmbares Salz einer solchen Verbindung, eines solchen Esters oder Amids, oder eine Verbindung, ein Ester, ein Amid oder ein Salz wie in Anspruch 7 definiert zur Verwendung als Pharmazeutikum.

9. Pharmazeutische Zusammensetzung umfassend eine Verbindung der Formel I, wie in Anspruch 1 dargestellt, worin R₁ bis R₇, R und X die in einem der Ansprüche 1 bis 6 angegebenen Bedeutungen haben oder ein physiologisch hydrolysierbarer und annehmbarer Ester davon oder ein physiologisch hydrolysierbares und annehmbares Amid davon oder ein pharmazeutisch annehmbares Salz einer solchen Verbindung, eines solchen Esters oder Amids, oder eine Verbindung, ein Ester, ein Amid oder Salz nach Anspruch 7 zusammen mit einem pharmazeutisch annehmbaren Lösungsmittel oder Träger.

## Revendications (Revendications pour l'(les) Etat(s) contractant(s) suivant(s): AT, BE, CH, DE, FR, GB, IT, LI, LU, NL, SE)

1. Un composé de formule I 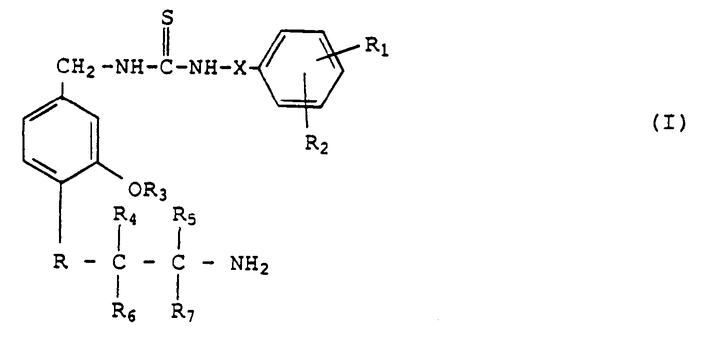 dans laquelle
l'un de R₁ et R₂ signifie un groupe alkyle en C₄ ramifié et l'autre signifie l'hydrogène ou un groupe alkyle en C₄ ramifié,
R₃ signifie l'hydrogène ou un groupe alkyle en C₁-C₄,
R₄ et R₅ signifient, indépendamment, l'hydrogène, un halogène ou un groupe alkyle en C₁-C₅, alkyle en C₁-C₅ substitué, aryle, aryle substitué ou non substitué ou un groupe de formule -COOH, -COOR₈ ou -CONR₉R₁₀ où R₈ signifie un groupe alkyle en C₁-C₅ et R₉ et R₁₀ signifient, indépendamment, l'hydrogène ou un groupe alkyle en C₁-C₅,
R₆ et R₇ ont, indépendamment, les significations données pour R₄ et R₅ ou forment, ensemble avec l'atome de carbone auquel ils sont fixés, un groupe cycloalkyle en C₃-C₇ substitué ou non substitué,
R signifie -O-, -S- ou -NH-, et
X signifie -(CH₂)ₙ- où n signifie 0, 1, 2 ou 3,
ou un ester ou un amide physiologiquement hydrolysable et physiologiquement acceptable de ce composé ou un sel pharmaceutiquement acceptable d'un tel composé, ester ou amide.

2. Un composé, ester, amide ou sel selon la revendication 1, dans lesquels, dans la formule I, R₁ signifie un groupe tert.-butyle et R₂ signifie l'hydrogène ou un groupe tert.-butyle.

3. Un composé, ester, amide ou sel selon la revendication 2, dans lesquels, dans la formule I, R₁ signifie un groupe tert.-butyle en position 3 et R₂ signifie un groupe tert.-butyle en position 5, ou bien R₁ signifie un groupe tert.-butyle en position 4 et R₂ signifie l'hydrogène.

4. Un composé, amide ou sel selon l'une quelconque des revendications 1 à 3, dans lesquels, dans la formule I, R₃ signifie un groupe méthyle, R₄, R₅, R₆ et R₇ signifient chacun l'hydrogène, R signifie -O- et X signifie -CH₂-.

5. Un composé ou un sel selon l'une quelconque des revendications 1 à 4.

6. La N-[4-(2-aminoéthoxy)-3-méthoxy-benzyl]-N¹-[3,5-di(tert.-butyl)benzyl]thiourée ou un sel d'addition d'acide pharmaceutiquement acceptable de ce composé.

7. La N-[4-(2-aminoéthoxy)-3-méthoxy-benzyl]-N¹-[4-tert.-butylbenzyl]thiourée ou un sel d'addition d'acide pharmaceutiquement acceptable de ce composé.

8. Un composé, ester, amide ou sel tels que définis à l'une quelconque des revendications 1 à 6, pour l'utilisation comme médicaments.

9. Un composé, ester, amide ou sel tels que définis à la revendication 7, pour l'utilisation dans le traitement de la douleur, des maladies inflammatoires ou des douleurs inflammatoires.

10. Une composition pharmaceutique comprenant un composé, ester, amide ou sel tels que définis à l'une quelconque des revendications 1 à 7, ensemble avec un diluant ou un véhicule pharmaceutiquement acceptables.

## Revendications (Revendications pour l'(les) Etat(s) contractant(s) suivant(s): ES, GR)

1. Un procédé de préparation d'un composé de formule I 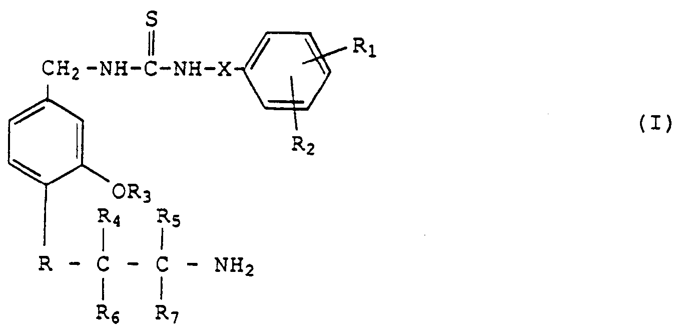 dans laquelle
l'un de R₁ et R₂ signifie un groupe alkyle en C₄ ramifié et l'autre signifie l'hydrogène ou un groupe alkyle en C₄ ramifié,
R₃ signifie l'hydrogène ou un groupe alkyle en C₁-C₄,
R₄ et R₅ signifient, indépendamment, l'hydrogène, un halogène ou un groupe alkyle en C₁-C₅, alkyle en C₁-C₅ substitué, aryle, aryle substitué ou non substitué ou un groupe de formule -COOH, -COOR₈ ou -CONR₉R₁₀ où R₈ signifie un groupe alkyle en C₁-C₅ et R₉ et R₁₀ signifient, indépendamment, l'hydrogène ou un groupe alkyle en C₁-C₅,
R₆ et R₇ ont, indépendamment, les significations données pour R₄ et R₅ ou forment, ensemble avec l'atome de carbone auquel ils sont fixés, un groupe cycloalkyle en C₃-C₇ substitué ou non substitué,
R signifie -O-, -S- ou -NH-, et
X signifie -(CH₂)ₙ- où n signifie 0, 1, 2 ou 3,
ou d'un ester ou d'un amide physiologiquement hydrolysable et acceptable de ce composé ou d'un sel pharmaceutiquement acceptable d'un tel composé, ester ou amide,
caractérisé en ce qu'il comprend
a) pour la préparation d'un composé de formule I, la déprotection d'un composé de formule I qui est sous forme amino protégée, ou
b) pour la préparation d'un ester ou d'un amide physiologiquement hydrolysable et physiologiquement acceptable d'un composé de formule I, l'estérification ou l'amidation d'un composé de formule I par réaction avec un agent d'acylation ou d'amidation appropriés,
et la récupération d'un composé de formule I, d'un ester ou d'un amide ainsi obtenus, sous forme libre ou sous forme d'un sel pharmaceutiquement acceptable.

2. Un procédé selon la revendication 1, dans lequel, dans la formule I, R₁ signifie un groupe tert.-butyle et R₂ signifie l'hydrogène ou un groupe tert.-butyle.

3. Un procédé selon la revendication 2, dans lequel, dans la formule I, R₁ signifie un groupe tert.-butyle en position 3 et R₂ signifie un groupe tert.-butyle en position 5, ou bien R₁ signifie un groupe tert.-butyle en position 4 et R₂ signifie l'hydrogène.

4. Un procédé selon l'une quelconque des revendications 1 à 3, dans lequel, dans la formule I, R₃ signifie un groupe méthyle, R₄, R₅, R₆ et R₇ signifient chacun l'hydrogène, R signifie -O- et X signifie -CH₂-.

5. Un procédé selon la revendication 1, dans lequel, dans la formule I,
R₁ signifie un groupe tert.-butyle en position 3,
R₂ signifie un groupe tert.-butyle en position 5,
R₃ signifie un groupe méthyle,
R₄, R₅, R₆ et R₇ signifient chacun l'hydrogène,
R signifie -O-, et
X signifie -CH₂-.

6. Un procédé selon la revendication 1, dans lequel, dans la formule I,
R₁ signifie un groupe tert.-butyle en position 4,
R₂ signifie l'hydrogène,
R₃ signifie un groupe méthyle,
R₄, R₅, R₆ et R₇ signifient chacun l'hydrogène,
R signifie -O-, et
X signifie -CH₂-.

7. Un composé de formule I telle qu'illustrée à la revendication 1, dans lequel R₁ à R₇, R et X ont les significations données à l'une quelconque des revendications 1 à 6 ou un ester ou un amide physiologiquement hydrolysable et physiologiquement acceptable de ce composé ou un sel pharmaceutiquement acceptable de ce composé, de cet ester ou de cet amide, lorsqu'il est préparé selon un procédé tel que spécifié à l'une quelconque des revendications 1 à 6.

8. Un composé de formule I telle qu'illustrée à la revendication 1, dans lequel R₁ à R₇, R et X ont les significations données à l'une quelconque des revendications 1 à 6 ou un ester ou un amide physiologiquement hydrolysable et physiologiquement acceptable de ce composé ou un sel pharmaceutiquement acceptable d'un tel composé, ester ou amide, ou un composé, ester, amide ou sel tels que définis à la revendication 7, pour l'utilisation comme médicaments.

9. Une composition pharmaceutique comprenant un composé de formule I telle qu'illustrée à la revendication 1, dans lequel R₁ à R₇, R et X ont les significations données à l'une quelconque des revendications 1 à 6 ou un ester ou un amide physiologiquement hydrolysable et physiologiquement acceptable de ce composé ou un sel pharmaceutiquement acceptable d'un tel composé, ester ou amide, ou un composé, ester, amide ou sel tels que définis à la revendication 7, ensemble avec un solvant ou un véhicule pharmaceutiquement acceptables.
